# EUROPEAN PATENT APPLICATION

(11) **EP 2 123 775 A1**
(43) Date of publication of application: **25.11.2009**
(21) Application number: 08156603.6
(22) Date of filing: 20.05.2008
(51) Int. Cl.: C12Q 1/68

(54) **Analysis for the genetic disposition for hip dysplasia in Canidae**

(71) Applicant: Stiftung Tierärztliche Hochschule Hannover, 30559 Hannover (DE)
(72) Inventor: Distl, Ottmar, Prof.Dr., 30559 Hannover (DE); Marschall, Yvonne, Dr., 31632 Husum-Bolsehle (DE); Stock, Kathrin-Friederike, Dr., 29352 Adelheidsdorf (DE)
(74) Representative: Taruttis, Stefan Georg

(57) **Abstract**

The invention provides an analytical method comprising the identification of genetic markers that are coupled to individual genetic factors which could be shown to be contributing to the HD phenotype. The analytical method is based on the determination of an aberration, e.g. a single nuclear polymorphism, in at least one genetic marker. Marker sequences relevant for the genetic disposition to develop HD are given as the wild-type sequences which determine a positive influence on hip development, i.e. no support of HD, whereas a mutant sequence determines a negative influence, i.e. an increase in phenotypic HD.

SEQUENCE LISTING

<110>  Stiftung Tierärztliche Hochschule Hannover

<120>  Genetic test for hip dysplasia in Canidae

<130>  T1006EP

<160>  69

<170>  PatentIn version 3.4

<210>  1
<211>  16
<212>  DNA
<213>  Artificial

<220>
<223>  Marker Seq.-ID No 1

<400>  1
caagagttcc agttcc                                                    16

<210>  2
<211>  20
<212>  DNA
<213>  Artificial

<220>
<223>  Marker Seq.-ID No 2

<400>  2
gcaatgcatc ggttgttttt                                                20

<210>  3
<211>  20
<212>  DNA
<213>  Artificial

<220>
<223>  Marker Seq.-ID No 3

<400>  3
accttaggta gtaccaaata                                                20

<210>  4
<211>  20
<212>  DNA
<213>  Artificial

<220>
<223>  Marker Seq.-ID No 4

<400>  4
tgtgagttga acatgtaaaa                                                20

<210>  5
<211>  21
<212>  DNA
<213>  Artificial

<220>
<223>  Marker Seq.-ID No 5

<400>  5

ttagaaaggt gactttccag g                                                21

<210>  6
<211>  23
<212>  DNA
<213>  Artificial

<220>
<223>  Marker Seq.-ID No 6

<400>  6
taagaatgag agtgtatttt gtc                                              23

<210>  7
<211>  20
<212>  DNA
<213>  Artificial

<220>
<223>  Marker Seq.-ID No 7

<400>  7
cttacctgcg tcccttcccc                                                  20

<210>  8
<211>  23
<212>  DNA
<213>  Artificial

<220>
<223>  Marker Seq.-ID No 8

<400>  8
caacattgtt acactaaaca ctg                                              23

<210>  9
<211>  23
<212>  DNA
<213>  Artificial

<220>
<223>  Marker Seq.-ID No 9

<400>  9
aattctcacc gaaagtctgc cag                                              23

<210>  10
<211>  22
<212>  DNA
<213>  Artificial

<220>
<223>  Marker Seq.-ID No 11

<400>  10
tggaaggaaa cacaggaggg aa                                               22

<210>  11
<211>  22
<212>  DNA
<213>  Artificial

<220>

<223>  Marker Seq.-ID No 11

<400>  11
ctaaaatctg acatagccaa ag                                                22


<210>  12
<211>  19
<212>  DNA
<213>  Artificial

<220>
<223>  Marker Seq.-ID No 12

<400>  12
gtacttggat gctgcatac                                                    19


<210>  13
<211>  21
<212>  DNA
<213>  Artificial

<220>
<223>  Marker Seq.-ID No 13

<400>  13
caaacacgtg atgtctttaa a                                                 21


<210>  14
<211>  22
<212>  DNA
<213>  Artificial

<220>
<223>  Marker Seq.-ID No 14

<400>  14
aggaaggaca gtgccttgcc ct                                                22


<210>  15
<211>  18
<212>  DNA
<213>  Artificial

<220>
<223>  Marker Seq.-ID No 15

<400>  15
gtggcagata tgagtcac                                                     18


<210>  16
<211>  19
<212>  DNA
<213>  Artificial

<220>
<223>  Marker Seq.-ID No 16

<400>  16
caggatggca ccccagttc                                                    19


<210>  17
<211>  22
<212>  DNA

<213>  Artificial

<220>
<223>  Marker Seq.-ID No 17

<400>  17
tagcttcctg aaataccatt at                                                22


<210>  18
<211>  17
<212>  DNA
<213>  Artificial

<220>
<223>  Mutant Marker Seq.-ID No 18

<400>  18
caagagtgtc cagttcc                                                      17


<210>  19
<211>  20
<212>  DNA
<213>  Artificial

<220>
<223>  Mutant Marker Seq.-ID No 19

<400>  19
gcaatgcatt ggttgttttt                                                   20


<210>  20
<211>  20
<212>  DNA
<213>  Artificial

<220>
<223>  Mutant Marker Seq.-ID No 20

<400>  20
accttaggta ttaccaaata                                                   20


<210>  21
<211>  20
<212>  DNA
<213>  Artificial

<220>
<223>  Mutant Marker Seq.-ID No 21

<400>  21
tgtgagttga acatgtaaaa                                                   20


<210>  22
<211>  21
<212>  DNA
<213>  Artificial

<220>
<223>  Mutant Marker Seq.-ID No  22

<400>  22
ttagaaaggt gactttccag g                                                 21

<210>  23
<211>  23
<212>  DNA
<213>  Artificial

<220>
<223>  Mutant Marker Seq.-ID No 23

<400>  23
taagaatgat agtgtatttt gtc                                              23


<210>  24
<211>  20
<212>  DNA
<213>  Artificial

<220>
<223>  Mutant Marker Seq.-ID No 24

<400>  24
cttacctgca tcccttcccc                                                  20


<210>  25
<211>  23
<212>  DNA
<213>  Artificial

<220>
<223>  Mutant Marker Seq.-ID No 25

<400>  25
caacattgtt atactaaaca ctg                                              23


<210>  26
<211>  23
<212>  DNA
<213>  Artificial

<220>
<223>  Mutant Marker Seq.-ID No 26

<400>  26
aattctcact gaaagtctgc cag                                              23


<210>  27
<211>  22
<212>  DNA
<213>  Artificial

<220>
<223>  Mutant Marker Seq.-ID No 27

<400>  27
tggaaggaac cacaggaggg aa                                               22


<210>  28
<211>  22
<212>  DNA
<213>  Artificial

<220>
<223>  Mutant Marker Seq.-ID No 28

<400>  28

ctaaaatctg acatagccaa ag                                                22

<210>  29
<211>  19
<212>  DNA
<213>  Artificial

<220>
<223>  Mutant Marker Seq.-ID No 29

<400>  29
gtacttggac gctgcatac                                                   19

<210>  30
<211>  21
<212>  DNA
<213>  Artificial

<220>
<223>  Mutant Marker Seq.-ID No 30

<400>  30
caaacacgtg acgtctttaa a                                                21

<210>  31
<211>  22
<212>  DNA
<213>  Artificial

<220>
<223>  Mutant Marker Seq.-ID No 31

<400>  31
aggaaggaca gcgccttgcc ct                                                22

<210>  32
<211>  18
<212>  DNA
<213>  Artificial

<220>
<223>  Mutant Marker Seq.-ID No 32

<400>  32
gtggcagatg tgagtcac                                                    18

<210>  33
<211>  19
<212>  DNA
<213>  Artificial

<220>
<223>  Mutant Marker Seq.-ID No 33

<400>  33
caggatggcg ccccagttc                                                   19

<210>  34
<211>  22
<212>  DNA
<213>  Artificial

<220>

<223>  Mutant Marker Seq.-ID No 34

<400>  34
tagcttcctg caataccatt at                                              22


<210>  35
<211>  17
<212>  DNA
<213>  Artificial

<220>
<223>  Mutant Marker Seq.-ID No 35

<400>  35
caagagtatc cagttcc                                                    17


<210>  36
<211>  20
<212>  DNA
<213>  Artificial

<220>
<223>  Primer Seq.-ID No 40

<400>  36
gcagcatacc tctaccatgc                                                 20


<210>  37
<211>  20
<212>  DNA
<213>  Artificial

<220>
<223>  Primer Seq.-ID No 41

<400>  37
aagcaataag gcaacaccac                                                 20


<210>  38
<211>  20
<212>  DNA
<213>  Artificial

<220>
<223>  Primer Seq.-ID No 42

<400>  38
accactttga gccttgtttg                                                 20


<210>  39
<211>  20
<212>  DNA
<213>  Artificial

<220>
<223>  Primer Seq.-ID No 43

<400>  39
gttcaacacc ctttgagcac                                                 20


<210>  40
<211>  22
<212>  DNA

<213>  Artificial

<220>
<223>  Primer Seq.-ID No 44

<400>  40
ttgaaaggca tttacatcag tg                                                22


<210>  41
<211>  20
<212>  DNA
<213>  Artificial

<220>
<223>  Primer Seq.-ID No 45

<400>  41
atcagcaatc caatctcagc                                                   20


<210>  42
<211>  20
<212>  DNA
<213>  Artificial

<220>
<223>  Primer Seq.-ID No 46

<400>  42
gagctccagc tctcaaacca                                                   20


<210>  43
<211>  20
<212>  DNA
<213>  Artificial

<220>
<223>  Primer Seq.-ID No 47

<400>  43
gccacatctg ggaagaagag                                                   20


<210>  44
<211>  20
<212>  DNA
<213>  Artificial

<220>
<223>  Primer Seq.-ID No 48

<400>  44
cccaccacca tcttgtcttc                                                   20


<210>  45
<211>  20
<212>  DNA
<213>  Artificial

<220>
<223>  Primer Seq.-ID No 49

<400>  45
tcccagctgg agatctctgt                                                   20

<210>  46
<211>  20
<212>  DNA
<213>  Artificial

<220>
<223>  Primer Seq.-ID No 50

<400>  46
gattggctta tttgcacgag                                                    20


<210>  47
<211>  20
<212>  DNA
<213>  Artificial

<220>
<223>  Primer Seq.-ID No 51

<400>  47
ttttcccttt tgtggttctg                                                    20


<210>  48
<211>  19
<212>  DNA
<213>  Artificial

<220>
<223>  Primer Seq.-ID No 52

<400>  48
atgtcctggc acaccaaag                                                     19


<210>  49
<211>  20
<212>  DNA
<213>  Artificial

<220>
<223>  Primer Seq.-ID No 53

<400>  49
acgtggtagc tggaggacag                                                    20


<210>  50
<211>  20
<212>  DNA
<213>  Artificial

<220>
<223>  Primer Seq.-ID No 54

<400>  50
atccctcttt gatgcatgtc                                                    20


<210>  51
<211>  21
<212>  DNA
<213>  Artificial

<220>
<223>  Primer Seq.-ID No 55

<400>  51

gagtattgtc aagggcctgt c                                              21

<210>  52
<211>  20
<212>  DNA
<213>  Artificial

<220>
<223>  Primer Seq.-ID No 56

<400>  52
tcgaagagcc aaattagagg                                                 20

<210>  53
<211>  20
<212>  DNA
<213>  Artificial

<220>
<223>  Primer Seq.-ID No 57

<400>  53
tgacagtggc cttatcactg                                                 20

<210>  54
<211>  20
<212>  DNA
<213>  Artificial

<220>
<223>  Primer Seq.-ID No 58

<400>  54
gcagcttcca ttacacttgg                                                 20

<210>  55
<211>  20
<212>  DNA
<213>  Artificial

<220>
<223>  Primer Seq.-ID No 59

<400>  55
tgttcaaggc ctctgttagc                                                 20

<210>  56
<211>  22
<212>  DNA
<213>  Artificial

<220>
<223>  Primer Seq.-ID No 60

<400>  56
tggttttgca cttagtctga tg                                              22

<210>  57
<211>  20
<212>  DNA
<213>  Artificial

<220>

<223>  Primer Seq.-ID No 61

<400>  57
aactccaggg agagaactgg                                                    20


<210>  58
<211>  20
<212>  DNA
<213>  Artificial

<220>
<223>  Primer Seq.-ID No 62

<400>  58
ttcccatgtg taaccctgtc                                                    20


<210>  59
<211>  21
<212>  DNA
<213>  Artificial

<220>
<223>  Primer Seq.-ID No 63

<400>  59
tcacctctta gggaaaggaa g                                                  21


<210>  60
<211>  20
<212>  DNA
<213>  Artificial

<220>
<223>  Primer Seq.-ID No 64

<400>  60
agatgggagg gttttgtttg                                                    20


<210>  61
<211>  20
<212>  DNA
<213>  Artificial

<220>
<223>  Primer Seq.-ID No 65

<400>  61
tgattcggtt ctctggtctc                                                    20


<210>  62
<211>  20
<212>  DNA
<213>  Artificial

<220>
<223>  Primer Seq.-ID No 66

<400>  62
aatggaagac gttctcatgc                                                    20


<210>  63
<211>  20
<212>  DNA

<213>  Artificial

<220>
<223>  Primer Seq.-ID No 67

<400>  63
aggggaaaga cagaaggaag                                                    20


<210>  64
<211>  20
<212>  DNA
<213>  Artificial

<220>
<223>  Primer Seq.-ID No 68

<400>  64
tgggcatcat cctgatttag                                                    20


<210>  65
<211>  20
<212>  DNA
<213>  Artificial

<220>
<223>  Primer Seq.-ID No 69

<400>  65
ttgaagaact tgtcccaagc                                                    20


<210>  66
<211>  19
<212>  DNA
<213>  Artificial

<220>
<223>  Primer Seq.-ID No 70

<400>  66
gcctctactg gagggtgtg                                                     19


<210>  67
<211>  20
<212>  DNA
<213>  Artificial

<220>
<223>  Primer Seq.-ID No 71

<400>  67
aactctgcag gtccagacac                                                    20


<210>  68
<211>  20
<212>  DNA
<213>  Artificial

<220>
<223>  Primer Seq.-ID No 72

<400>  68
caaggttatt cagccagcag                                                    20

<210>  69
<211>  22
<212>  DNA
<213>  Artificial

<220>
<223>  Primer Seq.-ID No 73

<400>  69
tcctcaatca aaatgaaatc ac                                                22

## Description

The present invention relates to a process for analysis of the genetic disposition in individuals of the genus Canidae, especially in Canis familiaris, i.e. dogs, and in Canis lupus, i.e. wolf, in relation to hip dysplasia, also termed dysplasia of the coxa. In accordance with the process being based on the genetic analysis, the invention also relates to the use of the relevant genetic markers in the analytical process, to the use of oligonucleotides which are specific for a DNA or RNA section containing a relevant marker, e.g. for use as primers in PCR amplification from genomic canine DNA, as well as to the markers and to the oligonucleotides themselves. Preferred dogs for the analytical process of the invention belong to or are related to the race of Rottweiler, Pinschers, especially to the group of closely related dog races consisting of German shepherd dog, Bernese mountain dog, Great Swiss mountain dog, Hovawart, German wire-haired pointer and boxer, as the occurrence of the markers of the invention and their coupling to HD could be shown especially for this group of dog races.

The analytical process of the invention is based on the detection of at least one genetic aberration in at least one genetic marker found to be indicative of the genetic disposition for hip dysplasia (HD). Further, the invention provides a process for the determination of an animal of the genus Canidae in respect of developing phenotypic HD, and in respect of its genetic value for breeding in respect of the probability of passing on to offspring the genetic disposition for HD, e.g. an analytical process for use in breeding for selection of an animal for breeding according to a calculated value indicating the genetic disposition for genetically passing on hip dysplasia to offspring.

### State of the art

To date, dogs are evaluated for hip dysplasia by X-ray analysis, i.e. phenotypically.

For an assessment of the genetic disposition for HD when selecting a dog for breeding, e.g. for evaluating the breeding value of the animal in respect of HD, the animal itself can be analysed phenotypically and, preferably, the family members of the dog are analysed, especially its parents and siblings, and, if available, its previous offspring. Accordingly, the phenotypic analysis of an individual dog or of its family members only indirectly allows to deduce the genetic disposition of the dog for HD, and of its breeding value in respect of HD.

From Distl, GKF-aktuell (May 2008), it is known that in dogs HD probably is inherited according to a multifactorial heredity, including a small number of genes having a high influence on the development of HD, as well as a larger number of genes with a smaller effect on the occurrence of HD. Without going into detail, Distl quotes the number of 261 microsatellite markers, which were closely coupled to genomic regions relevant for HD. Without detail for any marker or gene, a cumulative relation between an arbitrary genomic breeding value to the proportion of dogs suffering from HD is shown.

Hamann et al. in J. Anim. Breed. Genet. 120, 258-268 (2003) statistically relate the analytic results from radiographs of coxofemoral joints of German shepherd dogs to a number of breeding and environmental conditions and conclude that HD is a predominantly genetically caused disease in these dogs.

Janutta et al. in J. of Heredity, 97(I), 13-20 (2006) describe complex segregation analyses in German shepherd dogs and conclude that in German populations, major genes relevant to HD segregate while the HD phenotype also depends on polygenic effects.

Marschall and Distl in Mamm. Genome 18, 861-870 (2007) in a whole genome statistical analysis found a number of quantitative trait loci (QTL) associated with HD in German shepherd dogs.

### Objects of the invention

It is an object of the present invention to provide an analytical process for the determination of the predisposition of an individual canine to develop HD, and, on the basis of the genetic disposition of an individual canine, to estimate the genetic value of the individual canine for breeding in respect of HD.

### General description of the invention

The invention achieves the above-mentioned objects by providing an analytical method comprising the identification of genetic markers that are coupled to individual genetic factors which could be shown to be contributing to the HD phenotype. The analytical method is based on the determination of an aberration, i.e. mutation, e.g. a single nuclear polymorphism, in at least one genetic marker. Markers relevant for the genetic disposition to develop HD are given in a table below, which are given as the wild-type sequences which determine a positive influence on hip development, i.e. no support of HD, whereas a mutant sequence determines a negative influence, i.e. an increase in phenotypic HD. Exemplary mutant sequences for the markers are given below. In the case of the marker Seq.-ID No. 1 (TiHo1), the mutant sequence Seq.-ID No. 18 contains an insertion at the indicated location, whereas the other mutations, e.g. of Seq.-ID Nos. 2, 3, 6, and 8 to 17, are single base exchanges, of which examples are given in Seq.-ID Nos. 19, 20, 23, and 25 to 34, respectively.

Accordingly, the present invention as defined in claim 1 provides a process for analysis of at least one genetic marker, preferably of at least two markers, more preferably of 17 markers in the genome of an individual of the genus Canidae, herein also referred to as a canine.

In the analytical process, aberrations from the wild-type sequence of at least one of the markers of Seq.-ID Nos. 1 to 17 indicates a genome having an increased genetic propensity of the individual canine to pass the genetic traits for HD to its offspring, and to develop phenotypic HD itself.

In a preferred embodiment, the invention relates to the calculation of the propensity of the individual dog to develop phenotypic HD on the basis of a sum of marker specific numerical values for each of the analysed markers, preferably 2 to 17 markers. In the calculation of the genetic disposition for HD, each marker that is analysed is weighted to account for its individual influence on the development of an HD phenotype. For weighting of individual markers, the calculation of the genetic disposition of an individual canine to develop phenotypic HD is the result of an addition of a specific numerical value for each homozygous individual marker, also termed the additive effect of a marker, to a specific numerical value for each heterozygous individual marker, also termed the dominance effect of a marker. Accordingly, the calculation is based on generating the sum of marker specific numerical values of the markers analysed, preferably a numerical value for the markers found in the analytical process to be homozygous, provided with a positive prefix or algebraic sign (+) for the genotype of the mutant marker associated with HD and with a negative prefix (-) for the genotype of the marker not associated with HD, e.g. wild-type marker, and of a numerical value found in the analytical process to be heterozygous, provided with a positive prefix (+) for the genotype of the mutant marker associated with HD and with a negative prefix (-) for the genotype of the marker not associated with HD, e.g. wild-type marker.

The positive prefix or positive algebraic sign for aberrant markers, which are especially found to be associated with phenotypic HD, and the negative prefix or negative algebraic sign for wild-type markers are not contained in the table to follow and are introduced to the numeral when calculating the sum. The marker specific numerical values for the homozygous genotype of the marker and for the heterozygous genotype of the marker are given in the detailed description that follows. The marker specific numerical values for the marker specific homozygous genotype and for the heterozygous phenotype reflect their proportionate influences on phenotypic HD and therefore have a preferable relative proportion to one another. The absolute values of the marker specific numerical values can be changed as long as their relative proportions are essentially maintained.

The genetic disposition for HD, which is calculated as the sum of the individual numerical values specific for each of the tested homozygous and heterozygous markers, respectively, gives the genotypic numerical value for the genetic disposition for HD. By way of a correlation curve, the genotypic numerical value can be converted or assigned to the percentage of the risk for the animal to develop phenotypic HD. The risk of offspring of the analysed parental animal to develop phenotypic HD can be analysed by a determination of the possible genotypic marker set as derived from the parental animals, preferably assuming unrestricted recombination between the markers, and assigning a genotypic numerical value to the offspring recombinants. Accordingly, the invention provides a genotypic numerical value for the genetic disposition for HD, which numerical value is a suitable breeding value for an individual dog, allowing the evaluation of the relative risk for an individual canine animal to develop phenotypic HD, and to pass on the genetic traits for development of phenotypic HD, as well as to determine the risk for the offspring recombinants of a pairing to develop phenotypic HD. For a conversion of the genotypic numerical values into the percentage of the risk for HD, a standardisation is used prior to entering the value into one of the graphic relations between HD-risk and genotypic numerical value.

When comprising the assessment of offspring of an individual to develop phenotypical HD, or of the individual animal that is analysed to develop phenotypical HD itself, by assigning to the numerical value for the genetic disposition for HD to the percentage of phenotypic HD for the genotype, the analytical process in a preferred embodiment comprises the use of correlation curves representing the statistical relation between the numerical value for genetic HD disposition with the percentage of severe HD phenotype (HD-E), medium HD phenotype (HD-D), and slight HD phenotype (HD-C), respectively. Preferably, the correlation curve represents the statistical relation of the numerical value for genotypic HD disposition with all HD phenotypes, e.g. the occurrence of HD in any form.

Correlation curves are given below, wherein the X-axis shows the sum of the individual marker specific numerical values, i.e. the genotypic numerical value for HD, wherein the Y-axis gives the proportion of animals afflicted with phenotypic HD, separately for the sum of HD cases, for mild HD, for medium HD, and for severe HD. The relationship between the genotypic numerical values and the proportion of animals with phenotypic HD is given by the solid black line. The absolute values for the genotypic numerical values are in a one-to-one relation to the absolute numerical values for marker specific numerical values and can be changed proportionally when changing the absolute marker specific numerical values, e.g. by applying an identical multiplication factor.

It is a specific advantage of the methods of the invention that the disposition of a dog can be determined from analysing the individual alone, i.e. without a phenotypic analysis of the family of the individual. Further, the genetic analysis can be carried out on the basis of a biopsy sample, preferably a blood sample of an animal, avoiding the burden of X-ray exposure, while yielding an individual assessment of the genetic disposition for HD. One of the advantages of the invention is that it allows the detailed analysis of animals with indeterminate phenotypic HD (HD-B) to a phenotypic percentage of either an HD-free (HD-A) or an HD-afflicted (HD-C, HD-D, HD-E) phenotype.

### Detailed description of the invention

The invention is now described in greater detail with reference to the figures, showing the relation between the numerical value for the genotypic as determined as the weighted sum of marker specific numerical values for a set of 17 markers to the proportion of the genotypic disposition for phenotypic HD of an individual canine or of its offspring, namely in the solid black line in
- Figure 1 for all types of phenotypic HD as a total of HD-C, HD-D, and HD-E,
- Figure 2 for HD-C,
- Figure 3 for HD-D,
- Figure 4 for HD-E, and
- Figures 5 to 7, Figures 11 to 14, Figures 16 to18 and Figures 20 to 21 show electrophoresis gels of marker specific RFLP reactions,
- Figures 8 to 10, Figure 15, and Figure 19 show measurement results for marker specific real-time PCR analyses.

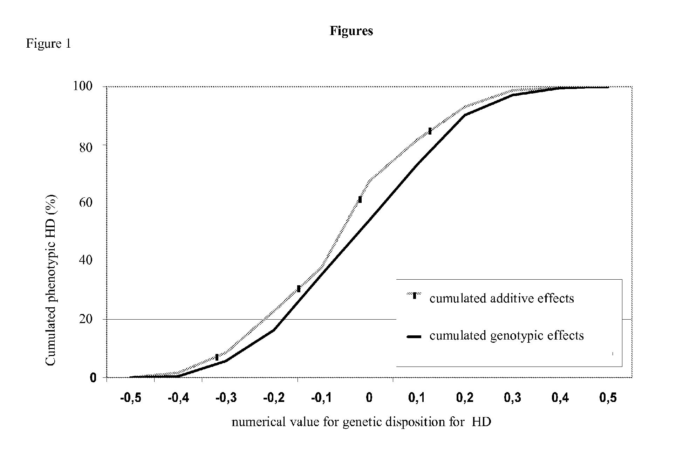

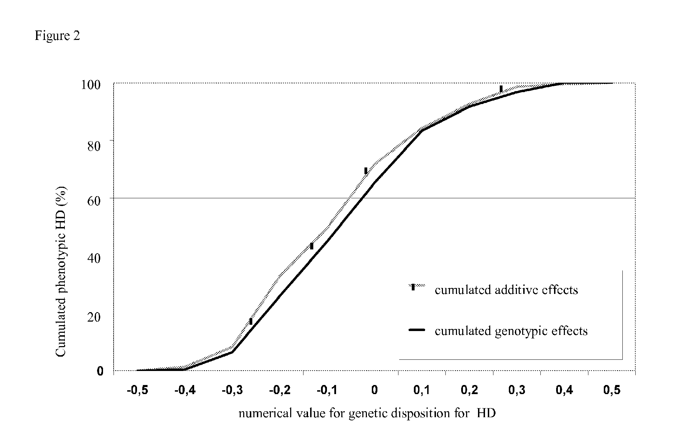

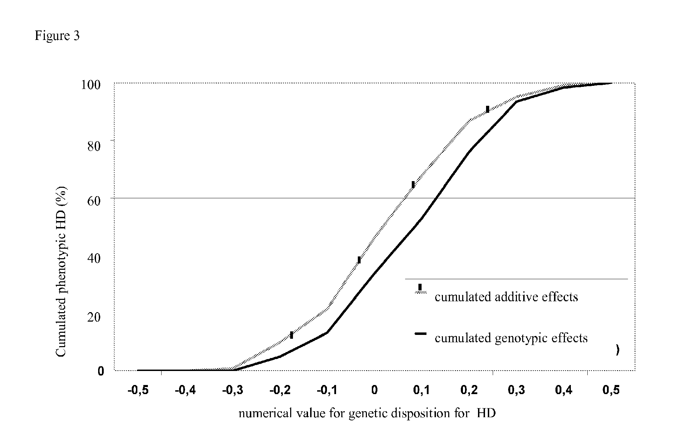

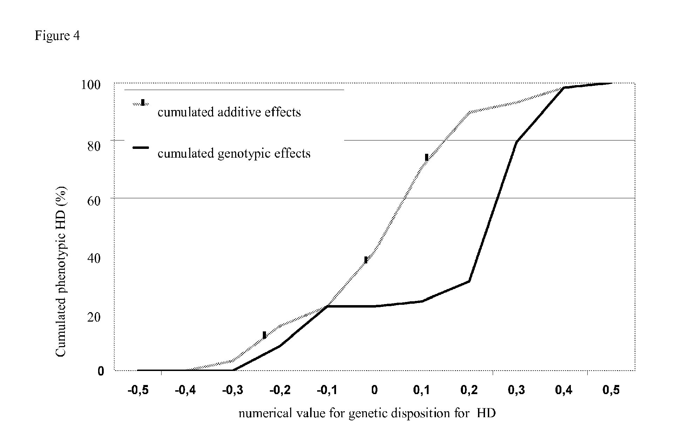

In Figures 1 to 4, the numerical value for genetic disposition for HD is standardized to a range of -0.5 to +0.5 by conversion of the sum of added marker specific numerical values as described below.

From a statistic analysis of canine populations, exemplified by dogs, the following genetic marker sequences could be identified, in which aberrations are linked to phenotypic HD. In the wild-type marker sequences, the nucleotide sequence of the markers are those not associated with HD, whereas mutations of the sequences have been found to be associated with the disposition for HD. Especially mutations of the nucleotide given in brackets or adjacent the nucleotide in brackets, e.g. insertions or deletions, have been found to be associated with HD; specific exemplary marker mutations which associated with the disposition for HD, especially of the nucleotide in brackets, are given in table 2.

**Table 1: Wild-type marker sequences**

| Seq.-ID | marker sequence 5' to 3' | CFA / gene name | locus |
|---|---|---|---|
| No. | | localisation | |
| 1 | CAAGAGT[]TCCAGTTCC | 1 / *OSTF1* | intron 3 |
| (TiHo1) | | LOC484161 | |
| 35 | CAAGAGT[A]TCCAGTTCC | | |
| (TiHo1a) | | | |
| 2 | GCAATGCAT[C]GGTTGTTTTT | 3 / *PGM2* | intron 8- |
| (TiHo5) | | LOC479116 | intron 10 |
| 3 | ACCTTAGGTA[G]TACCAAATA | 4 / *MYPN* | intron2 |
| (TiHo7) | | LOC489013 | |
| 4 | TGTGAGTT[G]AACATGTAAAA | 8 / B8_263 | intergenic |
| (TiHo34) | | (BICF2P1184132) | |
| 5 | TTAGAAAGGT[G]ACTTTCCAGG | 9 / *OLFM1* | intron 4 |
| (TiHo35) | | (BICF2S22937555) | |
| 6 | TAAGAATGA[G]AGTGTATTTTGTC | 19 / *ACVR2A* | exon 9/ |
| (TiHo9) | | LOC476140 | intron 9 |
| 7 | CTTACCTGC [G] TCCCTTCCCC | 26 / B26_243 | intergenic |
| (TiHo33) | | (BICF2P844355) | |
| 8 | CAACATTGTTA[C]ACTAAACACTG | 29 / *PXMP3* | intron 1 |
| (TiHo12) | | LOC487007 | |
| 9 | AATTCTCAC[C]GAAAGTCTGCCAG | 32 / *IBSP* | exon 4- |
| (TiHo16) | | LOC609146 | intron 5 |
| 10 | TGGAAGGAA[A]CACAGGAGGGAA | 33 / *EPHA3* | UTR 5' |
| (TiHo18) | | LOC487930 | |
| 11 | CTAAAATCTGA[C]ATAGCCAAAG | 33 / *EPHA6* | intron 2-intron 3 |
| (TiHo19) | | LOC607935 | |
| 12 | GTACTTGGA[T]GCTGCATAC | 33 / *ABI3BP* | intron 3 |
| (TiHo20) | | LOC478544 | |
| 13 | CAAACACGTGA[T]GTCTTTAAA | 33 / *PCNP* | intron 2 |
| (TiHo21) | | LOC478546 | |
| 14 | AGGAAGGACAG[T]GCCTTGCCCT | 34 / *TRIO* | intron 12-intron 13 |
| (TiHo23) | | LOC478610 | |
| 15 | GTGGCAGAT[A]TGAGTCAC | 34 / *SEMA5A* | intron 3 |
| (TiHo24) | | LOC478622 | |
| 16 | CAGGATGGC[A]CCCCAGTTC | 34 / *SLC6A3* | exon 12/ |
| (TiHo25) | | LOC609304 | intron 12 |
| 17 | TAGCTTCCTG[A]AATACCATTAT | 34 / *FGF12* | exon 2/ intron 2 |
| (TiHo26) | | LOC478676 | |

The markers of Table 1 are unique or could be amplified uniquely from the isolated total canine DNA in a PCR amplificate using the marker specific primers given below.

For Seq.-ID No. 1 (TiHo1), the allele associated with phenotypic HD was identified in German shepherd dogs as an insertion of G at the site indicated in empty brackets, whereas an A inserted at the site indicated by brackets in Seq.-ID No. 1, indicated as Seq.-ID No. 35, in German shepherd dogs at least in some individuals was not associated with HD. In boxers, the wild-type allele, i.e. the non-HD-afflicted boxer allele is Seq.-ID No 1. For the purposes of the invention, the HD-associated mutant allele carries an insertion, whereas the non-HD allele of the marker is given as Seq.-ID No. 1.

The gene given for each marker, respectively, can presently considered as arbitrary and does not necessarily restrict the occurrence of the marker to the specific gene. The same applies to the locus, which is given for the marker in the gene that is named.

Exemplary marker sequences containing aberrations which have been found as being associated with HD are given in the following table 2, wherein the HD-associated mutation is identified by brackets [].

**Table 2: Marker sequences with mutations coupled to phenotypic HD**

| Seq.-ID | mutant marker sequence 5' to 3' | CFA / gene name | locus |
|---|---|---|---|
| No. | | localisation: HD-associated | |
| | | mutation | |
| 18 | CAAGAGT[G]TCCAGTTCC | 1 / *OSTF1* | intron 3 |
| (TiHo1-mut) | | LOC484161:g.7691_7692insA/G | |
| 19 | GCAATGCAT[T]GGTTGTTTTT | 3 / *PGM2* | intron 8- |
| (Tio5-mut) | | LOC479116:g.21668C>T | intron 10 |
| 20 | ACCTTAGGTA[T]TACCAAATA | 4 / *MYPN* | intron2 |
| (TiHo7-mut) | | LOC489013:g.35837T>G | |
| 21 | TGTGAGTT[G]AACATGTAAAA | 8 / B8_263 (BICF2P1184132) | intergenic |
| (TiHo34-mut) | | | |
| 22 | TTAGAAAGGT[G] ACTTTCCAGG | 9 / *OLFMI* (BICF2S22937555) | intron 4 |
| (TiHo35-mut) | | | |
| 23 | TAAGAATGA[T]AGTGTATTTTGTC | 19 / *ACVR2A* | exon 9/ |
| (TiHo9-mut) | | LOC476140:g.72096T>G | intron 9 |
| 24 | CTTACCTGC [A] TCCCTTCCCC | 26 / B26_243 (BICF2P844355) | intergenic |
| (TiHo33-mut) | | | |
| 25 | CAACATTGTTA[T]ACTAAACACTG | 29 / *PXMP3* | intron 1 |
| (TiHo 12-mut) | | LOC487007:g.5654T>C | |
| 26 | AATTCTCAC[T]GAAAGTCTGCCAG | 32 / *IBSP* | exon 4- |
| (TiHo16-mut) | | LOC609146:g.3158C>T | intron 5 |
| 27 | TGGAAGGAA[C]CACAGGAGGGAA | 33 / *EPHA3* | UTR 5' |
| (TiHo18-mut) | | LOC487930:g.115884A>C | |
| 28 | CTAAAATCTGA[C]ATAGCCAAAG | 33 / *EPHA6* | intron 2- |
| (TiHo19-mut) | | LOC607935:g.2901T>C | intron 3 |
| 29 | GTACTTGGA[C]GCTGCATAC | 33 / *ABI3BP* | intron 3 |
| (TiHo20-mut) | | LOC478544:g.60932C>T | |
| 30 | CAAACACGTGA[C]GTCTTTAAA | 33 / *PCNP* | intron 2 |
| (TiHo21-mut) | | LOC478546:g.7346T>C | |
| 31 | AGGAAGGACAG[C]GCCTTGCCCT | 34 / *TRIO* | intron 12- |
| (TiHo23-mut) | | LOC478610:g.90754C>T | intron 13 |
| 32 | GTGGCAGAT[G]TGAGTCAC | 34 / *SEMA5A* | intron 3 |
| (TiHo24-mut) | | LOC478622:g.62478A>G | |
| 33 | CAGGATGGC[G]CCCCAGTTC | 34 / *SLC6A3* | exon 12/ |
| (TiHo25-mut) | | LOC609304:g.29157A>G | intron 12 |
| 34 | TAGCTTCCTG[C]AATACCATTAT | 34 / *FGF12* | exon 2/ |
| (TiHo26-mut) | | LOC478676:g.41474A>C | intron 2 |

| | | | |
|---|---|---|---|
| CFA: Canis familiaris autosome | | | |

From Tables 1 and 2, the following single nucleotide polymorphisms of the marker sequences having relevance for HD can be identified with the genotypes associated with HD in their homozygous state, i.e. SNPs of both allels HD-associated:

**Table 3: exemplary SNP motives and HD-associated homozygous genotype:**

| Marker wild-type Seq.-ID No. | SNP-motive | HD-associated homozygosity |
|---|---|---|
| 1 (TiHo1) | insertion of G | GG |
| 2 (TiHo5) | C/T | TT |
| 3 (TiHo7) | T/G | TT |
| 4 (TiHo34) | A/G | GG |
| 5 (TiHo35) | G/C | GG |
| 6 (TiHo9) | T/G | TT |
| 7 (TiHo33) | G/A | AA |
| 8 (TiHo12) | T/C | TT |
| 9 (TiHo16) | C/T | TT |
| 10 (TiHo18) | A/C | CC |
| 11 (TiHo19) | T/C | CC |
| 12 (TiHo20) | C/T | CC |
| 13 (TiHo21) | T/C | CC |
| 14 (TiHo23) | C/T | CC |
| 15 (TiHo24) | A/G | GG |
| 16 (TiHo25) | A/G | GG |
| 17 (TiHo26) | A/C | CC |

The exemplary SNP motives, i.e. mutations of the markers relevant for HD were determined especially for pure-bred German shepherd dogs.

Aberrations can be determined in genomic DNA samples, preferably in DNA embodied by PCR amplificates comprising at least one marker, which PCR amplificates were generated using oligonucleotide primers specifically hybridising to a portion of genomic canine DNA comprising the marker. Within the DNA comprising markers, either in the form of genomic DNA or in the form of PCR amplificates, aberrations from the wild-type sequences of Seq.-ID Nos. 1 to 17 can be detected using standard procedures, e.g. hybridisation with a nucleic acid probe, by ligase chain reaction, PCR with detection of the specific amplification progress, e.g. real time PCR, and/or restriction fragment polymorphism (RFLP), each procedure being specific for detecting an aberration from one of the markers. Exemplary specific oligonucleotides for amplification of genomic DNA portions containing markers, e.g. DNA amplificates containing markers, are listed in following Table 4, wherein preferred methods for the detection of genetic aberrations in the markers are indicated. The oligonucleotides useful for generation of one amplificate are given pairwise as forward and backward primers.

**Table 4: Oligonucleotides for specific generation of amplificates containing markers from genomic canine DNA**

| Seq.-ID No. (name) sequences 5' to 3' | marker contained / | Tₐ | detection |
|---|---|---|---|
| forward primer | size of amplificate (bp) | | (enzyme, |
| backward primer | | | buffer, |
| | | | temp.) |
| 40 (TiHo1-1) | Seq.-ID No.1, No. 35, | 58 | RFLP: |
| GCAGCATACCTCTACCATGC | No. 18 | | (BciVI, |
| | (TiHo1 / TiHo1a / | | NEB4, |
| 41 (TiHo1-2) | TiHo1-mut) | | 37°C) |
| AAGCAATAAGGCAACACCAC | 527 | | |
| 42 (TiHo5-1) | Seq.-ID No.2, No. 19 | 57 | RFLP: |
| ACCACTTTGAGCCTTGTTTG | (TiHo5 / TiHo5-mut) | | (SfaNI, |
| | 587 | | NEB3, |
| 43 (TiHo5-2) | | | 37°C) |
| GTTCAACACCCTTTGAGCAC | | | |
| 44 (TiHo7-1) | Seq.-ID No.3, No. 20 | 58 | RFLP: |
| TTGAAAGGCATTTACATCAGTG | (TiHo7 / TiHo7-mut) | | (RsaI, |
| | 512 | | NEB1, |
| 45 (TiHo7-2) | | | 37°C) |
| ATCAGCAATCCAATCTCAGC | | | |
| 46 (TiHo34-1) | Seq.-ID No.4, No. 21 | 58 | ABI |
| GAGCTCCAGCTCTCAAACCA | (TiHo34 / TiHo34-mut) | | |
| | 470 | | |
| 47 (TiHo34-2) | | | |
| GCCACATCTGGGAAGAAGAG | | | |
| 48 (TiHo35-1) | Seq.-ID No.5, No. 22 | 60 | ABI |
| CCCACCACCATCTTGTCTTC | (TiHo35 / TiHo35-mut) | | |
| | 450 | | |
| 49 (TiHo35- | | | |
| 2)TCCCAGCTGGAGATCTCTGT | | | |
| 50 (TiHo9-1) | Seq.-ID No.6, No. 23 | 58 | ABI |
| GATTGGCTTATTTGCACGAG | (TiHo9 / TiHo9-mut) | | |
| | 576 | | |
| 51 (TiHo9-2) | | | |
| TTTTCCCTTTTGTGGTTCTG | | | |
| 52 (TiHo33-1) | Seq.-ID No.7, No. 24 | 62 | RFLP |
| ATGTCCTGGCACACCAAAG | (TiHo33 / TiHo33-mut) | | (BtsCI, |
| | 434 | | NEB4, |
| 53 (TiHo33-2) | | | 50°C) |
| ACGTGGTAGCTGGAGGACAG | | | |
| 54 (TiHo12-1) | Seq.-ID No.8, No. 25 | 58 | RFLP |
| ATCCCTCTTTGATGCATGTC | (TiHo 12 / TiHo 12-mut) | | (MaeIII, |
| | 589 | | MaeIII, |
| 55 (TiHo12-2) | | | 55°C) |
| GAGTATTGTCAAGGGCCTGTC | | | |
| 56 (TiHo16-1) | Seq.-ID No.9, No. 26 | 56 | RFLP |
| TCGAAGAGCCAAATTAGAGG | (TiHo16 / TiHo16-mut) | | (HphI, |
| | 542 | | NEB4, |
| 57 (TiHo16-2) | | | 37°C) |
| TGACAGTGGCCTTATCACTG | | | |
| 58 (TiHo18-1) | Seq.-ID No.10, No. 27 | 58 | RFLP |
| GCAGCTTCCATTACACTTGG | (TiHo18 / TiHo18-mut) | | (NlaIV, |
| | 503 | | NEB4, |
| 59 (TiHo18-2) | | | BSA) |
| TGTTCAAGGCCTCTGTTAGC | | | |
| 60 (TiHo19-1) | Seq.-ID No.11, No. 28 | 58 | ABI |
| TGGTTTTGCACTTAGTCTGATG | (TiHo 19 / TiHo 19-mut) | | |
| | 507 | | |
| 61 (TiHo19-2) | | | |
| AACTCCAGGGAGAGAACTGG | | | |
| 62 (TiHo20-1) | Seq.-ID No.12, No. 29 | 58 | RFLP |
| TTCCCATGTGTAACCCTGTC | (TiHo20 / TiHo20-mut) | | (HgaI, |
| | 502 | | NEBI, |
| 63 (TiHo20-2) | | | 37°C) |
| TCACCTCTTAGGGAAAGGAAG | | | |
| 64 (TiHo21-1) | Seq.-ID No.13, No. 30 | 56 | RFLP |
| AGATGGGAGGGTTTTGTTTG | (TiHo21 / TiHo21-mut) | | (Tsp45I, |
| | 577 | | NEBI, |
| 65 (TiHo22-2) | | | BSA, 65°C) |
| TGATTCGGTTCTCTGGTCTC | | | |
| 66 (TiHo23-1) | Seq.-ID No.14, No. 31 | 58 | RFLP |
| AATGGAAGACGTTCTCATGC | (TiHo23 / TiHo23-mut) | | (HaeII, |
| | 539 | | NEB4, |
| 67 (TiHo24-2) | | | BSA, 37°C) |
| AGGGGAAAGACAGAAGGAAG | | | |
| 68 (TiHo24-1) | Seq.-ID No.15, No. 32 | 58 | ABI |
| TGGGCATCATCCTGATTTAG | (TiHo24 / TiHo24-mut) | | |
| | 517 | | |
| 69 (TiHo24-2) | | | |
| TTGAAGAACTTGTCCCAAGC | | | |
| 70 (TiHo25-1) | Seq.-ID No.16, No. 33 | 58 | RFLP |
| GCCTCTACTGGAGGGTGTG | (TiHo25 / TiHo25-mut) | | (HhaI, |
| | 577 | | NEB4, |
| 71 (TiHo25-2) | | | BSA, 37°C) |
| AACTCTGCAGGTCCAGACAC | | | |
| 72 (TiHo26-1) | Seq.-ID No.17, No. 34 | 58 | RFLP |
| CAAGGTTATTCAGCCAGCAG | (TiHo26 / TiHo26-mut) | | (Hpy188III, |
| | 536 | | NEB4, |
| 73 (TiHo26-2) | | | BSA, 37°C) |
| TCCTCAATCAAAATGAAATCAC | | | |

| | | | |
|---|---|---|---|
| RFLP = restriction fragment length polymorphism of specific PCR amplificates using the indicated restriction enzyme, analysis by gel electrophoresis. ABI = 7300 real time PCR (Applied Biosystems, Darmstadt), a PCR process continously observing specific amplification using the indicated primers. | | | |

Preferably, the markers for which the analytical process is performed, are added to one numerical value as a measure for the genotype for the genetic propensity of the individual canine to develop phenotypical HD, including in the numerical value a weighting of each marker to reflect its influence. According to the invention, marker specific numerical values with the proportions of table 4 are used, which values are given and selected for each marker in the homozygous state and in the heterozygous state.

Preferably, DNA fragments or DNA amplificates obtained using PCR amplification on genomic DNA with the marker specific primers from Table 4 are analysed by RFLP or real-time PCR (ABI), i.e. using marker-specific oligonucleotides as primers for the PCR. PCR amplificates were obtained with the primer pairs of Table 4, which also gives sizes of amplificates. Examples for the analytical results are given in the following table, wherein for RFLP-analysis the respective specific SNP is given:

**Table 5: Results of genotyping in analysis of markers according to the invention**

| Marker Seq.-ID No. | Analysis | Fragment sizes (bp) or label of primer for nucleotide of allel | | |
|---|---|---|---|---|
| | | Homozygous wild-type allele | heterozygous | Homozygous for HD-associated allel |
| 1 (TiHo1) | RFLP | G: 527 | A/G: 102; 425; 527 | G: 102; 425 |
| 2 (TiHo5) | RFLP | T: 587 | C/T: 84, 503, 587 | C: 84, 503 |
| 3 (TiHo7) | RFLP | T: 111; 401 | G/T: 111; 118; 283; 401 | G: 111; 118; 283 |
| 4 (TiHo34) | ABI | G: FAM | | A: VIC |
| 5 (TiHo35) | ABI | G: FAM | | C: VIC |
| 6 (TiHo9) | ABI | T: VIC | | G: FAM |
| 7 (TiHo33) | RFLP | A: 150; 284 | A/G: 150; 284; 434 | G: 434 |
| 8 (TiHo12) | RFLP | T: 507; 82 | C/T: 248: 259; 507; 82 | C: 248; 259 |
| 9 (TiHo16) | RFLP | T: 258; 284 | C/T: 258; 284; 542 | C: 542 |
| 10 (TiHo18) | RFLP | C: 45; 83; 128; 229 | A/C: 45; 83; 128; 191; 229 | A: 83; 191; 229 |
| 11 (TiHo19) | ABI | C: FAM | | T: VIC |
| 12 (TiHo20) | RFLP | C: 134; 368 | C/T: 134; 368; 502 | T: 502 |
| 13 (TiHo21) | RFLP | C: 230; 347 | C/T: 230; 347; 577 | T: 577 |
| 14 (TiHo23) | RFLP | C: 206; 333 | C/T: 206; 333; 539 | T: 539 |
| 15 (TiHo24) | ABI | G: FAM | | A: VIC |
| 16 (TiHo25) | RFLP | G: 162; 415 | A/G: 162; 415; 577 | A: 577 |
| 17 (TiHo26) | RFLP | C: 536 | A/C: 105; 431; 536 | A: 105; 431 |

| | | | | |
|---|---|---|---|---|
| FAM and VIC (PCR-specific, obtainable from Applied Biosystems (ABI), Darmstadt) are dyes which are labels to the primer oligonucleotides. | | | | |

From following Table 6, a numerical value can be taken for each of the markers as detected in the homozygous state, i.e. only one duplicate allele of wild-type or aberrant (HD-associated) marker is detected, or in the heterozygous state, i.e. both one wild-type marker allele and one aberrant marker. In the case of homozygosity of aberrant markers, the numerical value is provided with a positive prefix (+), as a result adding to the numerical genotype for HD, whereas a detected wild-type marker is provided with a negative prefix (-) and as a result in the sum representing the genotype is subtracted, reducing the numerical genotype for HD.

**Table 6: Numerical values for weighting markers**

| Seq.ID No. | Marker | | relative risk for phenotypic HD | | | | numerical value | | | |
|---|---|---|---|---|---|---|---|---|---|---|
| | | | aberrant homozygous marker | | heterozygous marker | | homozygous marker | | heterozygous marker | |
| | | | mean (95% CI) | P | mean (95% CI) | P | mean (95% KI) | P | Mittelwert (95% KI) | P |
| 1, 18 | TiHo1 | (OSTF1_1) | 2.503 (1.577 - 3.971) | <0.001 | 1.703 (1.224 - 2.371) | 0.002 | 0.459 (0.228, 0.690) | <0.001 | 0.074 (-0.246, 0.394) | 0.652 |
| 2, 19 | TiHo5 | (PGM2_2) | 2.596 (1.638 - 4.112) | <0.001 | 1.554 (0.978 - 2.470) | 0.062 | 0.477 (0.247, 0.707) | <0.001 | -0.036 (-0.366, 0.294) | 0.830 |
| 3, 20 | TiHo7 | (MYPN_1) | 1.922 (1.216 - 3.036) | 0.005 | 1.412 (0.887 - 2.249) | 0.146 | 0.327 (0.098, 0.555) | 0.005 | 0.019 (-0.314, 0.352) | 0.912 |
| 4, 21 | TiHo34 | (B8_263) | 1.574 (0.764 - 3.242) | 0.219 | 1.044 (0.498 - 2.188) | 0.910 | 0.227 (-0.135, 0.588) | 0.219 | -0.184 (-0.626, 0.258) | 0.415 |
| 5, 22 | TiHo35 | (B9_499) | 2.892 (1.327 - 6.304) | 0.008 | 2.050 (1.181 - 3.559) | 0.011 | 0.531 (0.141, 0.921) | 0.008 | 0.187 (-0.467, 0.841) | 0.576 |
| 6, 23 | TiHo9 | (ACVR2A_2) | 2.186 (1.443 - 3.311) | <0.001 | 1.502 (1.049 - 2.149) | 0.026 | 0.391 (0.184, 0.599) | <0.001 | 0.016 (-0.287, 0.318) | 0.920 |
| 7, 24 | TiHo33 | (B26_243) | 4.232 (2.650 - 6.756) | <0.001 | 1.764 (1.087 - 2.862) | 0.022 | 0.721 (0.487, 0.955) | <0.001 | -0.154 (-0.504, 0.196) | 0.389 |
| 8, 25 | TiHo12 | (PXMP3_1) | 2.633 (1.246 - 5.564) | 0.011 | 1.924 (1.352 - 2.736) | <0.00 1 | 0.484 (0.110, 0.858) | 0.011 | 0.170 (-0.310, 0.650) | 0.487 |
| 9, 26 | TiHo16 | (IBSP_1) | 1.310 (0.756 - 2.270) | 0.335 | 1.067 (0.616 - 1.848) | 0.818 | 0.135 (-0.140, 0.410) | 0.335 | -0.071 (-0.425, 0.284) | 0.697 |
| 10, 27 | TiHo18 | (EPHA3_7) | 2.400 (1.574 - 3.658) | <0.001 | 1.537 (1.069 - 2.210) | 0.020 | 0.438 (0.227, 0.649) | <0.001 | -0.008 (-0.315, 0.299) | 0.961 |
| 11, 28 | TiHo19 | (EPHA6_3) | 6.281 (2.714 - 14.536) | <0.001 | 2.058 (1.439 - 2.943) | 1 | 0.919 (0.499. 1.338) | <0.001 | -0.197 (-0.716, 0.322) | 0.457 |
| 12, 29 | TiHo20 | (ABI3BP_1) | 1.255 (0.816 - 1.929) | 0.302 | 1.256 (0.882 - 1.788) | 0.206 | 0.113 (-0.102, 0.328) | 0.302 | 0.115 (0.191, 0.420) | 0.462 |
| 13, 30 | TiHo21 | (PCNP_4) | 4.887 (2.864 - 8.338) | <0.001 | 1.887 (1.114 - 3.197) | 0.018 | 0.793 (0.526, 1.060) | <0.001 | -0.158 (-0.507, 0.191) | 0.374 |
| 14, 31 | TiHo23 | (TRIO_1) | 13.498 (6.408 - 28.433) | <0.001 | 4.960 (3.484 - 7.062) | <0.00 1 | 1.301 (0.929, 1.674) | <0.001 | 0.300 (-0.163, 0.763) | 0.204 |
| 15, 32 | TiHo24 | (SEMA5A-1) | 2.839 (1.627 - 4.955) | <0.001 | 1.808 (1.034 - 3.162) | 0.038 | 0.522 (0.243, 0.800) | <0.001 | 0.070 (-0.289, 0.429) | 0.701 |
| 16 33 | TiHo25 | (SLC6A3_2) | 5.425 (2.835 - 10.379) | <0.001 | 3.263 (2.235 - 4.764) | <0.00 1 | 0.846 (0.521, 1.170) | <0.001 | 0.337 (-0.120, 0.795) | 0.149 |
| 34 | TiHo26 | (FGF12_1) | (2.4139 - 6.042) | <0.001 | (0.926 - 2.327) | 0.102 | (0.442, 0.899) | <0.001 | (-0.619, 0.046) | 0.091 |

| | | | | | | | | | | |
|---|---|---|---|---|---|---|---|---|---|---|
| CI = confidence intervall | | | | | | | | | | |

The values listed for the relative risk for HD show the relative influence for homozygous aberrant, i.e. HD-associated markers, and for the heterozygous marker.

### Example: Analysis of the genetic disposition for HD in a canine

As an example of a canine, German shepherd dogs were analysed according to the invention. From a blood sample of the dog, total DNA was isolated using ion exchange (QiaAmp Blood, Qiagen, Hilden). Reaction conditions are given below:

| **PCR** | |
|---|---|
| **reagent** | **volume per single reaction** |
| DNA | 1.5 µl |
| H₂O | 23 µl |
| Incubation Mix T.Pol with MgCl₂ [1.5 mM] | 3 µl |
| DMSO | 1 µl |
| forward primer (according to Table 3) | 1 µl |
| backward primer (according to Table 3) | 1 µl |
| dNTPs 5mM | 1 µl |
| Taq-DNA-polymerase 5 U/µl | 0.2 µl |
| **temperature programme** | |
| 4 min 94°C | 1 cycle |
| 30 s 94°C | 38 cycles |
| 30 s annealing temperature (according to Table 3) | |
| 45 s 72°C | |
| 10 min 4°C | 1 cyle |
| | |

| **RFLP** (restriction reaction over night) | |
|---|---|
| **reagent** | **volume per single reaction** |
| PCR product | 10 µl |
| H₂O | 5.4 µl oder 5.0 µl (incl. BSA) |
| buffer (according to Table 3) | 4.0 µl |
| bovine serum albumin (BSA) | 0.0 µl or 0.4 µl |
| endonuclease (according to Table 3) | 3 U |

Detection of the PCR reaction for real time PCR (Applied Biosystems) was optically during the reaction using fluorescence labels on at least one of the specific primers. For single base exchange mutations, e.g. for *ACVR2A*, *EPHA6, SEMA5A*, B8_263 and *OLFM1*, the following reaction conditions were used: for each reaction, 2.0 µl genomic DNA solution, 4.40 µl H₂O, 5.33 µl SensiMix DNA Kit, 0.27 µl Custom TaqMan^{®} SNP Genotyping Assays with 1 cycle of 95°C for 10 min, 40 cycles of 92°C for 15s and 60°C for 60s.

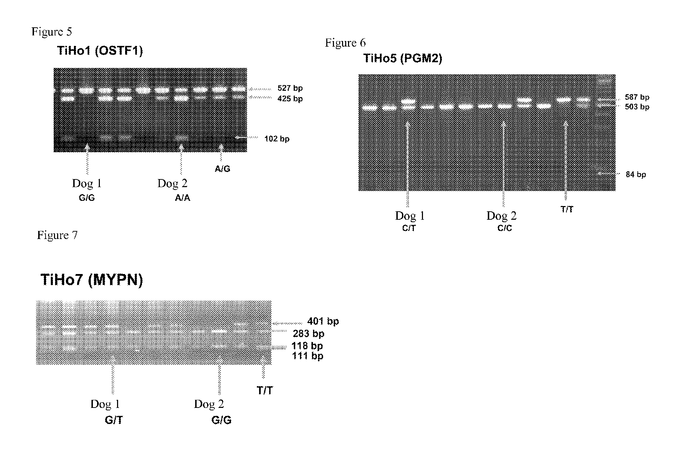

The analyses were carried out using the pairs of oligonucleotides given in Table 4 for PCR amplification, either in the real-time PCR system or followed by incubation with a marker specific restriction enzyme for RFLP analyses. In Figures 5 to 21 the respective measurement results are shown, i.e. for real-time PCR the relative fluorescence of labelled primers, and electrophoresis gels for RFLP analyses. For real-time PCR, exemplary results are shown in the figures, indicating that for each wild-type and mutant allele, the signal obtained differs depending on hybridisation of the primer. Preferably, analysis by RFLP and/or specific primer hybridisation in PCR, e.g. in real-time PCR, comprises the step of identifying the mutation by sequencing of the sequence section comprising the marker or mutant marker, respectively, to allow for assignment of signals from RFLP and/or PCR to HD-associated or wild-type alleles.

In the following discussion, N/N indicates the nucleotides of both allels in the marker positions indicated in brackets in Tables 1 and 2. References to the marker Seq.-ID Nos. refer to the wild-type, i.e. non-HD-associated allels, as the nucleotide indicating the wild-type allele or the HD-associated mutant allele is indicated in each case.

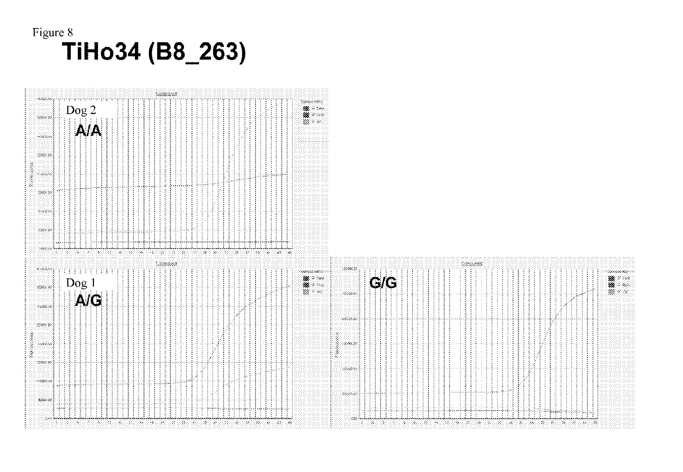

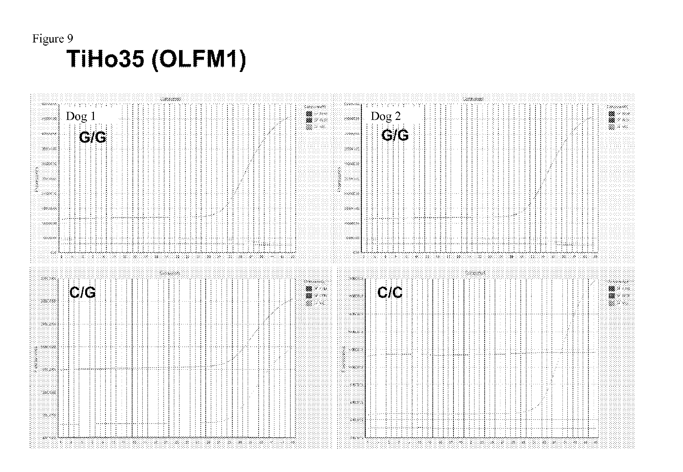

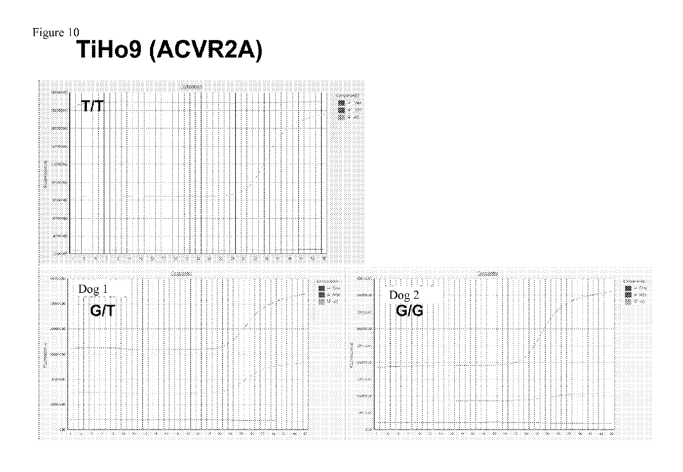

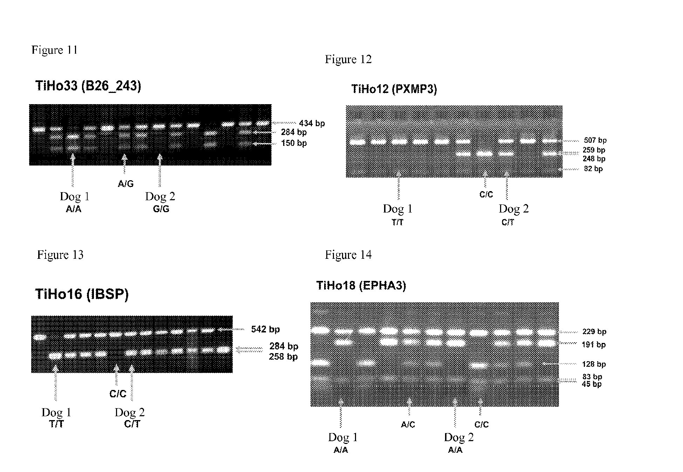

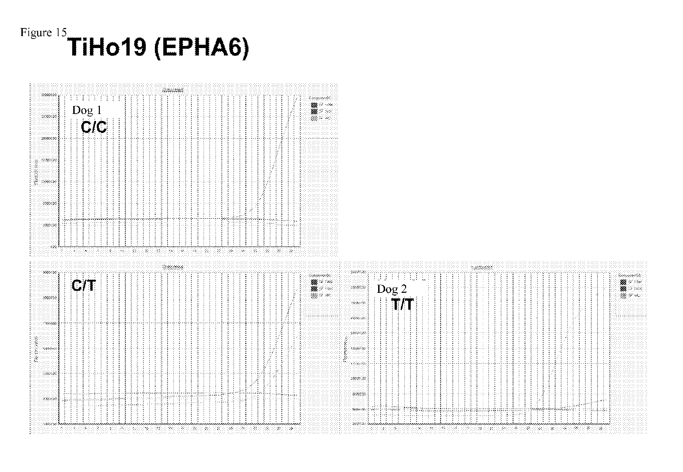

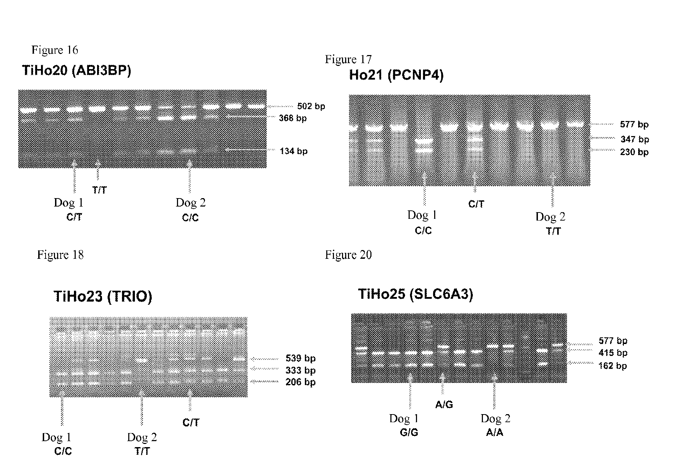

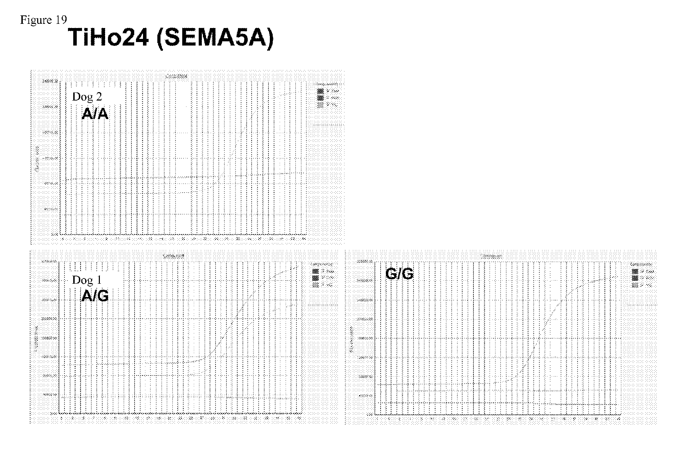

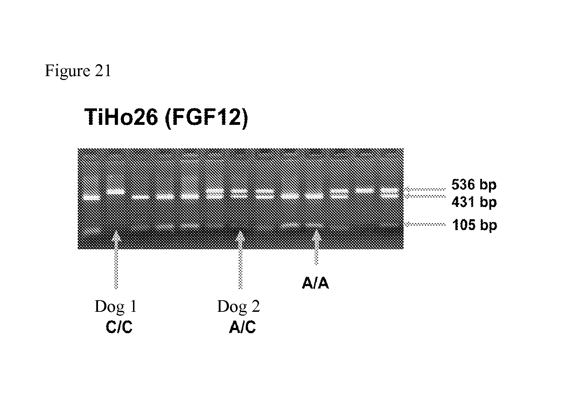

In detail, Figure 5 shows RFLP results for homozygote allels for dog 1 and dog 2 for marker Seq.-ID No. 1 with the indicated insertion mutations G/G, HD-associated for dog 1, and the non-HD allels A/A for dog 2. Figure 6 shows RFLP results for marker Seq.-ID No. 2, namely heterozygous (C/T) in dog 1 and non-HD associated allels C/C for dog 2. Further, as indicated by T/T, the HD-associated homozygous allels are found in another dog sample. Figure 7 shows heterozygous marker Seq.-ID No. 3 (G/T) for dog 1, and non-HD-associated homozygous allels G/G in dog 2. Figure 8 shows real-time PCR results for marker Seq.-ID No. 4, namely A/A for dog 2, A/G for dog 1, and for comparison a G/G sample of another dog. Figure 9 shows real-time PCR results for marker Seq.-ID No. 5, namely G/G for dogs 1 and 2, and for comparison samples known to be C/G and C/C, respectively. Figure 10 shows real-time PCR results for marker Seq.-ID No. 6, namely G/T for dog 1, G/G for dog 2, and a T/T homozygous sample for comparison. Figure 11 shows a gel electrophoresis of RFLP of marker Seq.-ID No. 7, namely for homozygous A/A dog 1, and G/G for dog 2, respectively, and a heterozygous sample A/G for comparison. Figure 12 shows dog 1 to be T/T homozygous for marker Seq.-ID No. 8, and dog 2 C/T heterozygous, a C/C homozygous sample was included for comparison. Figure 13 shows dog 1 to be T/T homozygous for marker Seq.-ID No. 9, and dog 2 C/T heterozygous with a C/C homozygous sample for comparison. Using RFLF analysis, Figure 14 shows dog 1 and dog 2 to be A/A homozygous for marker Seq.-ID No. 10, with a C/C homozygous sample and an A/C heterozygous sample for comparison. Figure 15 shows real-time PCR results for marker Seq.-ID No. 11, namely C/C for dog 1, T/T for dog 2, and a C/T heterozygous sample for comparison. Figure 16 shows dog 1 to be C/T heterozygous for marker Seq.-ID No. 12 and dog 2 C/C homozygous in comparison to a T/T homozygous sample. Figure 17 shows dog 1 to be C/C homozygous for marker Seq.-ID No. 13 and dog 2 T/T homozygous in comparison to a C/T heterozygous sample. Figure 18 shows dog 1 to be C/C homozygous for marker Seq.-ID No. 14 and dog 2 T/T homozygous in comparison to a C/T heterozygous sample. The real-time PCR of marker Seq.-ID No. 15 in Figure 19 shows dog 1 to be heterozygous (A/G) and dog 2 A/A homozygous in comparison to a G/G homozygous sample. Figure 20 shows dog 1 to be G/G homozygous for marker Seq.-ID No. 16, and dog 2 A/A homozygous in comparison to a heterozygous (A/G) sample. For marker Seq.-ID No. 17, Figure 21 shows dog 1 C/C homozygous and dog 2 heterozygous in comparison to an A/A homozygous sample.

For two German shepherd dogs the genotypes were detected and numerical values were assigned to the markers according to the respective homozygosity or heterozygosity as determined, as taken from Table 6. The numerical values for each marker, i.e. for homozygosity or heterozygosity as applicable of each marker were added to determine the sum of additive and dominance effects. The genomic breeding value for each dog was determined according to Figure 1. Dog 1 was determined to be of HD-A type, i.e. HD-free, whereas dog 2 was HD-D, i.e. affected by medium HD. Detailed results are given in the following table, wherein for each marker analysed the numerical value is given in the respective column for homozygous allels (additive effect, add), or for heterozygous allels (dominance effect, dom), and the non-applicable state of alleles is indicated by 0.

| marker | Dog 1 HD-associated alleles = 1 | | | Dog 2 HD-associated alleles = 12 | | |
|---|---|---|---|---|---|---|
| | genotype | add | dom | genotype | add | dom |
| 1 (TiHo1) | GG | -0.23 | 0 | AA | 0.23 | 0 |
| 2 (TiHo5) | CT | 0 | -0.10 | CC | 0.20 | 0 |
| 3 (TiHo7) | GT | 0 | 0.38 | GG | -0.10 | 0 |
| 4 (TiHo34) | AG | 0 | 0.06 | AA | 0.08 | 0 |
| 5 (TiHo35) | GG | -0.38 | 0 | GG | -0.38 | 0 |
| 6 (TiHo9) | GT | 0 | 0.04 | GG | 0.30 | 0 |
| 7 (TiHo33) | AA | -3.30 | 0 | GG | 0.30 | 0 |
| 8 (TiHo12) | TT | -0.18 | 0 | CT | 0 | 0.16 |
| 9 (TiHo16) | TT | -0.19 | 0 | CT | 0 | 0.16 |
| 10 (TiHo18) | AA | -0.08 | 0 | AA | 0.08 | 0 |
| 11 (TiHo19) | CC | -0.33 | 0 | TT | 0.33 | 0 |
| 12 (TiHo20) | CT | 0 | -0.02 | CC | 0.34 | 0 |
| 13 (TiHo21) | CC | -0.53 | 0 | TT | 0.53 | 0 |
| 14 (TiHo23) | CC | -0.91 | 0 | TT | 0.91 | 0 |
| 15 (TiHo24) | AG | 0 | -0.15 | AA | -0.01 | 0 |
| 16 (TiHo25) | GG | -0.02 | 0 | AA | 0.02 | 0 |
| 17 (TiHo26) | CC | -0.27 | 0 | AC | 0 | -0.14 |
| Genomic numerical value | | -3.05 | | | 3.02 | |

| | | | | | | |
|---|---|---|---|---|---|---|
| Genotypic numerical value = sum of add and dom values | | | | | | |

For using the graphic correlation of the resultant genotypic numerical value with the percentage of HD-phenotype of Figures 1 to 4, the genotypic numerical value needs to be converted by subtracting 0.03 and multiplying the result by a factor of 0.14, the conversion giving a standardized genotypic numerical value = 0.14 (genotypic numerical value - 0.33).

For dog 1, this gives 0.14 (-3.05 - 0.03) = -0.44, for dog 2 the standardisation gives 0.14 (3.02 - 0.03) = 0.43.

From Figure 1, solid black line, the numerical genotype value can be converted to a value of about 0 % for the cumulative risk for phenotypical HD for dog 1, and to a value of about 100% for dog 2.

In Figures 1 to 4, the line labelled with vertical dashes correlates the added standardised genotypic numerical values for additive effects of markers to respective HD percentages, i.e. without taking into account effects by heterozygous markers. The unlabelled line in these figures gives the relationship between the standardized genotypic numerical value and the risk for HD for the accumulated effects of homozygous markers (add) and heterozygous markers (dom).

## Claims

1. Process for analysis of the genetic disposition for hip dysplasia in a Canida, **characterized by** the detection of an aberration in at least one of the markers of Seq.-ID Nos. 1 to 17.

2. Process according to claim 1, **characterized in that**
Seq.-ID No. 1 or Seq.-ID No. 35 is contained in a PCR amplificate obtained by primers Seq.-ID No. 40 (TiHo1-1) and Seq.-ID No. 41 (TiHo1-2),
Seq.-ID No. 2 is contained in a PCR amplificate obtained by primers Seq.-ID No. 42 (TiHo5-1) and Seq.-ID No. 43 (TiHo5-2),
Seq.-ID No. 3 is contained in a PCR amplificate obtained by primers Seq.-ID No. 44 (TiHo7-1) and Seq.-ID No. 45 (TiHo7-2),
Seq.-ID No. 4 is contained in a PCR amplificate obtained by primers Seq.-ID No. 46 (TiHo34-1) and Seq.-ID No. 47 (TiHo34-2),
Seq.-ID No. 5 is contained in a PCR amplificate obtained by primers Seq.-ID No. 48 (TiHo35-1) and Seq.-ID No. 49 (TiHo35-2),
Seq.-ID No. 6 is contained in a PCR amplificate obtained by primers Seq.-ID No. 50 (TiHo9-1) and Seq.-ID No. 51 (TiHo9-2),
Seq.-ID No. 7 is contained in a PCR amplificate obtained by primers Seq.-ID No. 52 (TiHo33-1) and Seq.-ID No. 53 (TiHo33-2),
Seq.-ID No. 8 is contained in a PCR amplificate obtained by primers Seq.-ID No. 54 (TiHo12-1) and Seq.-ID No. 55 (TiHo12-2),
Seq.-ID No. 9 is contained in a PCR amplificate obtained by primers Seq.-ID No. 56 (TiHo16-1) and Seq.-ID No. 57 (TiHo16-2),
Seq.-ID No. 10 is contained in a PCR amplificate obtained by primers Seq.-ID No. 58 (TiHo18-1) and Seq.-ID No. 59 (TiHo18-2),
Seq.-ID No. 11 is contained in a PCR amplificate obtained by primers Seq.-ID No. 60 (TiHo19-1) and Seq.-ID No. 61 (TiHo19-2),
Seq.-ID No. 12 is contained in a PCR amplificate obtained by primers Seq.-ID No. 62 (TiHo20-1) and Seq.-ID No. 63 (TiHo20-2),
Seq.-ID No. 13 is contained in a PCR amplificate obtained by primers Seq.-ID No. 64 (TiHo21-1) and Seq.-ID No. 65 (TiHo21-2),
Seq.-ID No. 14 is contained in a PCR amplificate obtained by primers Seq.-ID No. 66 (TiHo23-1) and Seq.-ID No. 67 (TiHo23-2),
Seq.-ID No. 15 is contained in a PCR amplificate obtained by primers Seq.-ID No. 68 (TiHo24-1) and Seq.-ID No. 69 (TiHo24-2),
Seq.-ID No. 16 is contained in a PCR amplificate obtained by primers Seq.-ID No. 70 (TiHo25-1) and Seq.-ID No. 71 (TiHo25-2), and
Seq.-ID No. 17 is contained in a PCR amplificate obtained by primers Seq.-ID No. 72 (TiHo26-1) and Seq.-ID No. 73 (TiHo26-2).

3. Process according to one of the preceding claims, **characterized in that**
the aberration of Seq.-ID No. 1 or 35 is Seq.-ID No. 18,
the aberration of Seq.-ID No. 2 is Seq.-ID No. 19,
the aberration of Seq.-ID No. 3 is Seq.-ID No. 20,
the aberration of Seq.-ID No. 4 is Seq.-ID No. 21,
the aberration of Seq.-ID No. 5 is Seq.-ID No. 22,
the aberration of Seq.-ID No. 6 is Seq.-ID No. 23,
the aberration of Seq.-ID No. 7 is Seq.-ID No. 24,
the aberration of Seq.-ID No. 8 is Seq.-ID No. 25,
the aberration of Seq.-ID No. 9 is Seq.-ID No. 26,
the aberration of Seq.-ID No. 10 is Seq.-ID No. 27,
the aberration of Seq.-ID No. 11 is Seq.-ID No. 28,
the aberration of Seq.-ID No. 12 is Seq.-ID No. 29,
the aberration of Seq.-ID No. 13 is Seq.-ID No. 30,
the aberration of Seq.-ID No. 14 is Seq.-ID No. 31,
the aberration of Seq.-ID No. 15 is Seq.-ID No. 32,
the aberration of Seq.-ID No. 16 is Seq.-ID No. 33, and
the aberration of Seq.-ID No. 17 is Seq.-ID No. 34.

4. Process according to one of the preceding claims, **characterized in that** for the markers in the analysis, a sum of the genotypic genetic disposition for hip dysplasia is generated by adding marker specific numerical values which have the following numerical relation:
for the homozygous marker 0.459, or for the heterozygous marker 0.074 for Seq.-ID No. 1 or No. 35,
for the homozygous marker 0.477, or for the heterozygous marker -0.036 for Seq.-ID No. 2,
for the homozygous marker 0.327, or for the heterozygous marker 0.019 for Seq.-ID No. 3,
for the homozygous marker 0.227, or for the heterozygous marker -0.184 for Seq.-ID No. 4,
for the homozygous marker 0.531, or for the heterozygous marker 0.187 for Seq.-ID No. 5,
for the homozygous marker 0.391, or for the heterozygous marker 0.016 for Seq.-ID No. 6,
for the homozygous marker 0.721, or for the heterozygous marker -0.154 for Seq.-ID No. 7,
for the homozygous marker 0.484, or for the heterozygous marker 0.170 for Seq.-ID No. 8,
for the homozygous marker 0.135, or for the heterozygous marker -0.071 for Seq.-ID No. 9,
for the homozygous marker 0.438, or for the heterozygous marker -0.008 for Seq.-ID No. 10,
for the homozygous marker 0.919, or for the heterozygous marker -0.197 for Seq.-ID No. 11,
for the homozygous marker 0.113, or for the heterozygous marker 0.115 for Seq.-ID No. 12,
for the homozygous marker 0.793, or for the heterozygous marker -0.158 for Seq.-ID No. 13,
for the homozygous marker 1.301, or for the heterozygous marker 0.300 for Seq.-ID No. 14,
for the homozygous marker 0.522, or for the heterozygous marker 0.070 for Seq.-ID No. 15,
for the homozygous marker 0.846, or for the heterozygous marker 0.337 for Seq.-ID No. 16, and
for the homozygous marker 0.671, or for heterozygous markers -0.287 for Seq.-ID No. 17, wherein for markers without aberration, a positive prefix is applied and for markers showing an aberration, a negative prefix is applied.

5. Process according to claim 4, **characterized in that** the sum of marker specific numerical values is converted to a percentage for the genetic disposition for hip dysplasia of the genotype according to the relation of Figure 1 for the cumulative genetic disposition for hip dysplasia, Figure 2 for the genetic disposition for slight hip dysplasia, Figure 3 for the genetic disposition for medium hip dysplasia, and/or Figure 4 for the genetic disposition for severe hip dysplasia, wherein the numerical values of the X-axis are entered into the Figures following conversion to standardized genotypic numerical values by subtracting from the sum of marker specific numerical values the value of 0.03, and multiplication of the subtraction result by a factor of 0.14.

6. Process according to one of the preceding claims, **characterized by** selecting an individual Canida for breeding.

7. Genetic marker for analysis of the genetic disposition for hip dysplasia in a Canida in a process according to one of the preceding claims, which genetic marker comprises a sequence of the group of Seq.-ID Nos. 1 to 35.

8. Oligonucleotide for use as a primer for synthesis of an amplificate comprising a genetic marker according to claim 7, selected from
Seq.-ID No. 40 (TiHo5-1), Seq.-ID No. 41 (TiHo5-2),
Seq.-ID No. 42 (TiHo5-1), Seq.-ID No. 43 (TiHo5-2),
Seq.-ID No. 44 (TiHo7-1), Seq.-ID No. 45 (TiHo7-2),
Seq.-ID No. 46 (TiHo34-1), Seq.-ID No. 47 (TiHo34-2),
Seq.-ID No. 48 (TiHo35-1), Seq.-ID No. 49 (TiHo35-2),
Seq.-ID No. 50 (TiHo9-1), Seq.-ID No. 51 (TiHo9-2),
Seq.-ID No. 52 (TiHo33-1), Seq.-ID No. 53 (TiHo33-2),
Seq.-ID No. 54 (TiHo12-1), Seq.-ID No. 55 (TiHo12-2),
Seq.-ID No. 56 (TiHo16-1), Seq.-ID No. 57 (TiHo16-2),
Seq.-ID No. 58 (TiHo18-1), Seq.-ID No. 59 (TiHo18-2),
Seq.-ID No. 60 (TiHo19-1), Seq.-ID No. 61 (TiHo19-2),
Seq.-ID No. 62 (TiHo20-1), Seq.-ID No. 63 (TiHo20-2),
Seq.-ID No. 64 (TiHo21-1), Seq.-ID No. 65 (TiHo21-2),
Seq.-ID No. 66 (TiHo23-1), Seq.-ID No. 67 (TiHo23-2),
Seq.-ID No. 68 (TiHo24-1), Seq.-ID No. 69 (TiHo24-2),
Seq.-ID No. 70 (TiHo25-1), Seq.-ID No. 71 (TiHo25-2),
Seq.-ID No. 72 (TiHo26-1), Seq.-ID No. 73 (TiHo26-2).

9. Oligonucleotide according to claim 8, **characterized in** the pairwise use of the primers:
Seq.-ID No. 40 (TiHo5-1) in combination with Seq.-ID No. 41 (TiHo5-2),
Seq.-ID No. 42 (TiHo5-1) in combination with Seq.-ID No. 43 (TiHo5-2),
Seq.-ID No. 44 (TiHo7-1) in combination with Seq.-ID No. 45 (TiHo7-2),
Seq.-ID No. 46 (TiHo34-1) in combination with Seq.-ID No. 47 (TiHo34-2),
Seq.-ID No. 48 (TiHo35-1) in combination with Seq.-ID No. 49 (TiHo35-2),
Seq.-ID No. 50 (TiHo9-1) in combination with Seq.-ID No. 51 (TiHo9-2),
Seq.-ID No. 52 (TiHo33-1) in combination with Seq.-ID No. 53 (TiHo33-2),
Seq.-ID No. 54 (TiHo12-1) in combination with Seq.-ID No. 55 (TiHo12-2),
Seq.-ID No. 56 (TiHo16-1) in combination with Seq.-ID No. 57 (TiHo16-2),
Seq.-ID No. 58 (TiHo18-1) in combination with Seq.-ID No. 59 (TiHo18-2),
Seq.-ID No. 60 (TiHo19-1) in combination with Seq.-ID No. 61 (TiHo19-2),
Seq.-ID No. 62 (TiHo20-1) in combination with Seq.-ID No. 63 (TiHo20-2),
Seq.-ID No. 64 (TiHo21-1) in combination with Seq.-ID No. 65 (TiHo21-2),
Seq.-ID No. 66 (TiHo23-1) in combination with Seq.-ID No. 67 (TiHo23-2),
Seq.-ID No. 68 (TiHo24-1) in combination with Seq.-ID No. 69 (TiHo24-2),
Seq.-ID No. 70 (TiHo25-1) in combination with Seq.-ID No. 71 (TiHo25-2),
Seq.-ID No. 72 (TiHo26-1) in combination with Seq.-ID No. 73 (TiHo26-2).
